# EUROPEAN PATENT SPECIFICATION

(11) **EP 1 300 471 B1**
(45) Date of publication and mention of the grant of the patent: **12.09.2007**
(21) Application number: 01947123.4
(22) Date of filing: 29.06.2001
(51) Int. Cl.: C12Q 1/04, C12R 1/05, C12R 1/19, C12R 1/22, C12R 1/37, C12R 1/385, C12R 1/42

(54) **NUTRITIONAL MIXTURE AND METHOD FOR EARLY IDENTIFICATION AND COUNT OF GRAM-NEGATIVE ORGANISMS**
NÄHRMIXTUR UND VERFAHREN ZUR FRÜHINDENTIFIZIERUNG UND ZÄHLUNG VON GRAMNEGATIVEN ORGANISMEN
MELANGE NUTRITIF ET PROCEDE D'IDENTIFICATION ET DE DENOMBREMENT RAPIDE D'ORGANISMES GRAM-NEGATIFS

(30) Priority: 29.06.2000 CU 1602000
(43) Date of publication of application: 09.04.2003
(73) Proprietor: CENTRO NACIONAL DE BIOPREPARADOS, Provincia La Habana 6048 (CU)
(72) Inventor: TSORAEVA, Anna, Provincia La Habana (CU); RODRIGUEZ MARTINEZ, Claudio, Provincia La Habana (CU); QUESADA MUNIZ, Vivian de Jesus, Provincia La Habana (CU)
(74) Representative: Prins, Adrianus Willem
(86) International application number: PCT/CU2001/000004
(87) International publication number: WO 2002/000921

(56) References cited:
- WO-A-96/30543
- ES-A- 2 079 319
- US-A- 5 194 374
- DATABASE MEDLINE [Online] 1990 CHANG G ET AL: "Tryptophan and galactoside (TAG) media: simple and specific ways to enumerate E. coli and total coliforms in water and food" XP002901967

## Description

### Technical sector

The present invention is related to the field of Microbiology and particularly with a nutritive mixture and a procedure for the identification and of the differential and early count of Gram-negative organisms.

Several techniques for the detection and count of target microorganisms, for example of E. *coli* and coliform organisms, are extensively used in microbiological quality control of different products (water, foods, etc.). Nevertheless, the studies are continued to achieve the most simple and effective procedures.

Generally, very few biochemical characteristics are known that can be attributed only to one specific genus of Gram-negative bacteria, so the use of different sets of appropriate tests are needed to achieve a true diagnosis.

Actually, there is a wide range of culture techniques for the detection and differentiation of different groups of Gram-negative bacteria; some of them are qualitative and other quantitative ones.

Violet Red Bile Agar incorporates bile salts and crystal violet, which inhibit Gram-positive bacteria (Soria Melquizo, F. Manual Difco. Décima edición. 1984; Manual MERCK de Medios de Cultivo. 1990; Manual de Medios de Cultivo OXOID. 1995). This medium contains also lactose and as pH indicator, neutral red. Coliform organisms grow on the medium and ferment the lactose. The neutral red provides the intense red color to the colony and to the surrounding zone of the medium. There are necessary additional confirmative tests for a sure differentiation of *E. coli* and coliform organisms, as for example culturing in the Brilliant Green Broth with lactose or streaking in the Eosine Methylene Blue Agar (Soria Melquizo, F. Manual Difco. Décima edición. 1984; Manual MERCK de Medios de Cultivo. 1990; Manual de Medios de Cultivo OXOID. 1995). Although, the test results are not known until after 48 h of incubation of the sample.

On the other hand, there is a group of solid *Salmonella* detecting culture media, which use, as least, two biochemical characteristics of this genus, that are evident by the presence or absence of colony and culture media colors. Among them can be mentioned: Desoxycholate Citrate Agar, Hektoen Enteric Agar, S. S. Agar, XLD Agar, Christensen Agar and Brilliant Green Agar (Soria Melquizo, F. Manual Difco. Décima edición. 1984; Manual MERCK de Medios de Cultivo. 1990; Manual de Medios de Cultivo OXOID. 1995).

These media generally contain inhibitors of Gram-positive and of some Gram-negative bacteria, for example, bile salts, sodium citrate and brilliant green. *Salmonella* detection is based on the ability to ferment one or more carbohydrates and to produce the hydrogen sulfide in the presence of sodium thiosulphate and an iron salt. In practice, these culture media are not very specific, because several species (*Proteus vulgaris, P. mirabilis, Citrobacter freundii*) can develop colorless colonies with black center, characteristic of *Salmonella.* Moreover, the H₂S production by *Salmonella* strains does not happen always, due to several factors, such as the pH of the medium and the oxygen concentration around the colonies, which can influence this biochemical reaction.

Alain Rambach, in 1993, patented a medium for the identification of *Salmonella* (U.S. Patent No. 5 194 374), which includes the use of a chromogenic substrate that develops a blue color, as result of the hydrolysis carried out by β-galactosidase enzyme, and the degradation of the propylene glycol. The main biochemical test used in this medium for the *Salmonella* detection consists of the propylene glycol fermentation, which is visually detected by a color change of the pH indicator- neutral red.

The product allows the differentiation only of coliform bacteria and *Salmonella* non-*typhi* from *Proteus* and other Gram-negative bacteria. Moreover, in practice approximately 9% of the *Salmonella* strains can show β-galactosidase enzyme activity and about 30% of them do not ferment the propylene glycol (Kaluzewski y Tomczuk, Med. Dosw. Mikrobiol., 1995, 47: 155-168).

Roth and collaborators received the approval for the U.S. Patent No. 5 393 662, in which the author proposed a medium and a method for the identification and count of *E. coli* and coliforms applying substrates with different chromophore groups. This medium does not allow the identification and count of *E. coli* O157:H7, *Salmonella* and other non-coliform enterobacteria. Mean while, *Shigella sonnei* causes false positive results since it develops colonies with the same chromatic characteristics as *E. coli.*

In 1995 was approved a patent application of Monget et al (U.S. Patent No. 4 277 561), for a method for the bacteriological analysis, and a medium for the detection of *Salmonella*. This method is based on the specific capacity of *Salmonella* to ferment glucuronic acid or one of its salts and to not produce β-galactosidase enzyme. The mentioned medium includes nutrients, a fluorogenic or chromogenic substrate for β-galactosidase, glucuronic acid or one of its salts and a pH indicator. This culture medium contains, also, inhibitory substances for non-*Salmonella* genera (brilliant green and sodium desoxycholate), which limits the ability to detect other Gram-negative organisms and disables its use for the enumeration of microorganisms. The medium includes sodium glucuronate as a supplement after sterilization. According to Denis and collaborators, the specificity of this culture medium is of 93,3%, while for the other traditional *Salmonella* detecting medium (Hektoen Enteric Agar) it is about 85,3% (Denis, et to the, Revue francaise des laboratoires, Décembre 1994, No. 271).

Rambach applied a patent for a culture medium and a method for the detection of enteroahemorrhagic strains of *E. coli* (WO Patent Application No. 97/39103). The invention consisted on a selective culture medium for the differentiation of *E. coli,* particularly of 0157 and/or O11 serotypes. The medium contains a chromogenic substrate for α-galactosidase enzyme. To increase the differentiating ability of the method, other chromogenic substrates have been added, especially for β-glucosidase, which characterizes a great number of coliform bacteria, and for β-glucuronidase, which is characteristic for *E. coli*, except O157 and O11 serogroups. However, this culture medium does not allow the differentiation of other Gram-negative bacteria and, on the other hand, it has been reported that some *E. coli* and *Citrobacter* strains can produce false-positive results (Wallace and Jones, J. Appl. Bacteriol., 1996, 81: 663-668).

Roth and collaborators, in March 1998 received the approval of the U.S. patent No. 5,726,031. In the patent is described a test medium and a quantitative method for the identification and differentiation of biological materials in a test sample. The method consists of the use of one chromogenic substrate, which is specific to the first biological material and produces the first color; other chromogenic substrate that is specific to the second biological material and produces the second color and a third biological material is able to split one of these substrates. The first and second biological materials are able to degrade one sugar, and the third biological material does not split this sugar. Moreover, the composition of the medium includes a pH indicator that changes the color of the medium around the colonies, colored by chromogenic substrates, when the sugar is degraded.

The main ingredients are 6-chloro-3-indolyl galactoside, 5-bromo-4-chloro-3-indolyl glucuronide, sorbitol and phenol red. Other compounds are bile salts, sodium lauryl sulfate, sodium desoxycholate, polyglycol ether, antibiotics and achriflavine derivatives.

The medium should include in its formulation an inductor of the enzymatic reactions: the isopropyl-β-D-thiogalactopyranoside.

There are included agars, pectins, carrageenans, alginates, xanthin, among other gelling agents, and peptones. This prototype can be considered the nearest to the present invention and it shows a group of inconveniences:
- The medium allows the identification and enumeration of *E. coli, E. coli* O157:H7 and the coliforms, but it is difficult to release a suitable identification of *Salmonella* and even impossible for some strains, because they may develop white color, as it occurs for other Gram-negative bacteria, such as *Proteus*. In the case of coexisting different species, it is difficult to detect the presence of the yellow zone around the colony, which is characteristic for *Salmonella,* since it is produced by culture medium acidification.
- *Salmonella typhi* cannot be differentiated from non-*typhi*.
- The coliforms cannot be differentiated in the medium, and it is necessary to apply other additional diagnostic media for the further identification of very important pathogens, such as *Klebsiella.*
- The medium does not allow the identification and count of non-coliform Gram-negative bacteria, such as *Pseudomonas.*
- The employed method needs from 24 to 48 hours to perform the identification and count of claimed microorganisms, so this makes it as slow as the traditional culturing methods.
- The composition of the medium became more complex, expensive and unstable due to the inclusion of sodium dodecyl sulfate, acriflavine and/or antibiotics. It occurs, mainly, by the presence of antibiotics that must be added as a supplement, because they are heat sensible and cannot be added to the powdered dehydrated formulation.
- The growth favoring ingredients are not enough; on one's own, to permit the early development (before 24 hours) of the reactions that allow the identification of the microorganisms. The medium even needs a β-galactosidase inductor, as IPTG.
- The method provides the reactions to take place up to 40 °C, rather than 44 °C, established in the traditional methods. This fact requires the establishing of a new parameter in routine equipment or techniques.
- The routine microbiological analysis procedures do not foresee the incubation temperature of 40 °C, so an extensive validation of the method and its approval as an official method is necessary.

### Disclosure of the invention.

The objective of the present invention consists is to provide a nutrient mixture and a procedure for the identification and early enumeration of Gram-negative organisms.

The innovation of the present invention consists of, for the first time, providing a nutrient mixture, for the identification and early count of Gram-negative organisms and the procedure, in which said mixture is used, based on the appearance of at least five different colors, at the visible light, and of three fluorescent color emissions in the colonies; halos of three different colors and a zone of precipitation around such colonies and/or the combination of all those elements and changes in culture medium color.

Novel elements are contributed, providing sufficient quantities of tryptophan. This essential amino acid takes part in a significant number of metabolic reactions, serving as basis for the identification procedures. These quantities are supplied, taking into account their relationship with the organic and inorganic salts and with the color and/or fluorescence providing substances. The purpose of this mixture is to provide the early appearance of clear reactions.

New and unexpected elements were evidenced, when certain target organisms developed non-described colors, or have fluorescent emissions of previously not reported colors, or show dark zones around the colony (halo) of colors no described before, as for example, *Enterobacter* (green greyish colony center and yellowish fluorescence), *Citrobacter freundii* (dark violet colony with blue halo), *Salmonella* non *typhi* (center and yellow fluorescence after 24 h), *Salmonella typhi* (surrounding colony zone with opaque precipitate).

The relationships among the components of the mixture were established in an experimental way, and constitute the elements by which a new mixture is warranted. This relationship of compounds previously had never been described.

The present invention shows the following advantages:
- The whole identification and differentiation of the target organisms occur only in the presence of the prepared mixture, and there is no need of additional tests or culture media.
- The composition allows the growth of Gram-negative bacteria, since it not contains inhibitors of this group, and also allows the identification of most clinically and/or sanitarily important species, such as *E. coli, Salmonella, Pseudomonas, Klebsiella*, and also, the enumeration of *E. coli* and coliform organisms.
- The mixture offers security for the identification of different organisms, because it is carried out combining several reactions that are happened simultaneously. These reactions are based on the metabolic specificities of these organisms, and take place due to the appropriated compounds proportions of the mixture. Some of these reactions have been reported for the first time and may be considered as new findings, such as in the case of *Enterobacter* (*E. aerogenes, E. agglomerans, E. cloacae*), which appears as a rose to red colored colony with green greyish center; and *Salmonella* non-*typhi*, that appears as a red or red with yellowish center colony The both genera emit a yellowish fluorescence (after 24 hours of incubation). These characteristics allow the unequivocal identification of the organisms.
- The composition allows the identification and early count of a wide range of Gram-negative organisms. It allows the identification of *E. coli*, and the differentiation of *Salmonella typhi* from non-*typhi*, and of coliform organisms of sanitary relevance and other non-coliform Gram-negative bacteria, such as *Pseudomonas*. All of these organisms can be differentiated with the same one procedure, in the same dish and during maximally 22 hours.
- The identification is carried out mainly by the combined visualization of the appearance of five different colony colors, of three different fluorescence colors, the halos of three different colors around the colonies, and the precipitation opaque zone surrounding the colonies. The medium color changes can be of a secondary importance, or even not play any roll at all, in the identification of certain genera, such as the case of *Shigella sonnei, E. coli* O157:H7, *Pseudomonas aeruginosa*. This fact diminishes the risk of a false identification of different organisms in samples contaminated with different germs.
- The composition is very simple in its preparation; it does not require autoclaving, neither the addition of supplements that increase the risk of the sample contamination.
- Due to avoiding the sterilization in an autoclave, the nutrients and growth factors are better preserved and the pH value of the composition is kept more stable.
- The nutrients and growth factors incorporated in the mixture, mainly those rich on tryptophan, have such relationship of its absolute quantities in the composition, that can warrant the occurrence of early biochemical and chromatic reactions, in most of the cases before 22 hours.
- The nature of solid ingredients, conforming the mixture layers, allows concentration of the fluorescent emanations in the colony surrounding zone and do not diffuse to the rest of the dish. The mixture allows the secure count of fluorescent organisms, since the fluorescence does not diffuse to the surface of the medium.
- The selection of the Gram-positive organism growth inhibitors, their absolute quantities and the relationship with other components allows the appropriate growth of the target organisms, while the total inhibition (growth absence) of Gram-positive organisms is achieved.
- The wide range of substances, precursors or substrates for enzymatic reactions allows other reactions to take place and also allows the further identification of other Gram-negative organisms, when a third layer with indicators or revelators of reactions are added to the composition in a portion of the dish.
- The diagnostic specificity of the mixture was of 100% for the tested organisms and the analytical sensibility reached 10⁻⁶ CFU/mL starting from a an standardized suspension to 50 % of transmittance.
- The balance among nutrients and inhibitors allows the early identification and the exuberant growth of the target organisms, even of those that grow generally after 24 hours, as *Salmonella.*
- The results can be easily analyzed by a unspecialized personnel, because the identification or differentiation are not based on the morphological characteristics of the organisms.

### Detailed disclosure of the invention.

The present invention provides a nutrient mixture for the identification and early counting of Gram-negative organisms, which contains the following essential components:
- a mixture of tryptophan rich protein fractions or free tryptophan, which is in quantities from 22 to 46 % of the total mixture (weight/weight);
- a mixture of selected organic and/or inorganic salts in quantities from 15 to 20 % of the total mixture (weight/weight);
- a mixture of substances providing color or fluorescence in quantities from 0,3 to 37 % of the total mixture (weight/weight);
- inhibitor substances of the Gram-positive organisms in quantities from 2 to 4,5 % of the total mixture (weight/weight); and
- a mixture of substances providing solid structure to the medium, in quantities from 20 to 50 %.

The proportion of each one of the components of the mixture varies, within the predetermined ranges, in dependence of the nutritive medium that is wanted to prepare.

In the nutritive mixture of the invention, the content of the amino acid tryptophan, in the mixture of rich protein fractions, is in quantities from 0,25 to 3,8 % of said mixture.

The mixture of the present invention also contains a mixture of organic and/or inorganic salts, which are selected from the group consisting of NaCl, K₂HPO₄, KH₂PO4, (NH₄)₂SO₄, Na₂CO₃, and sodium piruvate and their mixtures, preferably been selected NaCI and Na₂CO₃.

In said mixture of the organic and inorganic salts the following quantities (weight/weight) in respect to the total mixture are provided:
- NaCl from 7 to 18 %;
- K₂HPO₄ from 6 to 11%;
- KH₂PO4 from 2 to 5 %;
- (NH₄)₂SO₄ from 1 to 4 %;
- Na₂CO₃ from 0,1 to 0,4 %; and
- sodium piruvate from 0,7 to 3 %.

Other component of nutrient mixture of the invention the mixture of substances that provide the color or fluorescence appearance, which are selected from the group consisting of X-GAL, MUG, sorbitol and neutral red, been selected preferably X-GAL y MUG. These substances are contained in the mixture in the following quantities (weight/weight) in respect to the total mixture:
- X-GAL from 0,16 to 0,36 %;
- MUG from 0,16 to 0,18 %;
- sorbitol from 15 to 36,5 %; and
- neutral red from 0,09 to 0,11 %.

On the other hand the mixture also contains inhibitor substances of the Gram-positive organisms, among them can be used sodium desoxycholate and bile salts.

Finally, the nutrient mixture of the invention contains a mixture of substances providing solid structure to the culture medium when the microorganisms grow to be tested, which can comprise the following combinations and amounts (weight/weight) in respect to the total mixture:
- agarose and agaropectina in quantities from 19 to 48 %, in combination with cellulose nitrate in quantities from 0,1 to 0,4 %; or
- cellulose and hemi cellulose in quantities from 1,4 to 3 %, in combination with cellulose nitrate in quantities from 0,1 to 0,4 %, or
- agarose and agaropectina only, in quantities from 19 to 48 %.

The nutrient mixture of the invention, once prepared has pH from 6,6 to 7,2

Another aspect of the invention is that it provides a procedure for the identification and early count of Gram negative organisms, wherein the nutritive mixture once solidified and contacted with the microorganisms or samples containing them, it is incubated for a period from 12 to 22 hours, at a temperature from 30 to 45 °C, from which it is possible the identification of said microorganisms at first sight, while fluorescence detection is carried out under ultraviolet light from 360 to 366 nm.

The procedure of the invention allows the identification of the microorganisms by the appearance of five different colors of the colonies, of fluorescent emissions of three colors, of halos of three colors, of color changes in the medium, and by the appearance of a precipitation zones surrounding the colonies, as well as the combinations of these characteristics.

Using the procedure of the invention, the identification of the organisms to detect is carried out as follows:
*Shigella sonnei-* By the appearance of blue greenish colonies with blue fluorescence and the orange medium;
*Shigella flexneri -* By the appearance of pale rose colonies, yellowish halo and the medium color is orange;
*Enterobacter* (*E. aerogenes, E. cloacae, E .agglomerans*) - By the appearance of pink to red colonies with green greyish center, yellow fluorescence and the red medium;
*Escherichia coli*, except verotoxigenic strains - By the appearance of light violet colonies, blue fluorescence and red medium;
*Escherichia coli* O157:H7 - By the appearance of blue greenish colonies and orange medium;
*Citrobacter freundii* - By the appearance of dark violet colonies with blue halo and red medium;
*Klebsiella pneumoniae* - By the appearance of light violet colonies and red medium;
*Salmonella typhi -* By the appearance of red colonies and red medium with a zone of opaque precipitate;
*Salmonella* "no typhi" - By the appearance of red colonies and red medium; after 24 h of incubation the color of the center becomes yellow and shows a yellow fluorescence;
*Pseudomonas aeruginosa -* By the appearance of pale rose colonies, greenish fluorescence before the 24 h of incubation and orange medium; greenish brown colonies and greenish surrounding zone, greenish fluorescence after the 24 h of incubation;
*Proteus, Providencia, Alcaligenes* and other Gram-negative organisms - By the appearance of colorless or transparent colonies and orange medium.

In a particular embodiment of the procedure of the invention, when using X-gal and MUG as sole components of a mixture that provide the color or fluorescence, the identification of total coliforms is made by the blue greenish color of the colonies, and specifically for *E. coli,* also by its blue fluorescence.

To conform the nutrient mixture there are carried out the following operations:

The substances, which provide tryptophan-rich protein fractions and/or free tryptophan in quantities from 22 to 46 %, are weighed in a conical flask, taking in account that the mixture should contain from 0.25 to 3,8 % of this amino acid, regarding to its dry weight. This flask should have a double nominal volume of the nutrient mixture to be prepared.

Subsequently the group of organic and inorganic salts is weighed and added to the previous mixture, in quantities between 15 and 20 % regarding the dry weight of the nutritive mixture.

This group of organic and inorganic salts are selected in the next, relative to the weight of the dehydrated nutritive mixture, quantities: NaCl from 7 to 18 %, K₂HPO₄ from 6 to 11 %, KH₂PO₄ from 2 to 5 %, (NH₄)₂SO₄ from 1 to 4 %, Na₂CO₃ from 0,1 to 0,4 % and sodium pyruvate from 0,7 to 3 %.

Then the inhibitors of Gram-positive organisms are weighed and added in quantities from 2 to 4,5 %, among them can be used sodium desoxycholate and bile salts.

Separately, in the analytic balance, the pre-mixture of the substances, that provide the color or fluorescence appearance, is weighed, in quantities from 0,3 to 37 %.

The pre-mixture should contain sorbitol in quantities from 15 to 36,5 %, MUG -from 0.16 to 0,18 %, X-GAL - from 0.16 to 0,36 % and neutral red - from 0.09 to 0,11 %, in relation to the dry weight of the nutritive mixture.

All of the previously mentioned components are mixed thoroughly with distilled or deionized water to obtain a homogeneous solution with the solute concentration from 3.5 to 6.5% and pH range from 6.6 to 7.2.

Finally the prepared solution is added to the agarose and agaropectin mixture and/or is poured on the structures formed by different forms of the cellulose and hemi cellulose mixtures.

If the first mixture agarose and agaropectin is added to the prepared solution, the formed suspension is mixed and allowed to soak for at least 15 min. Then it is heated until boiling, cooled down until approximately 45 °C and dispensed in the final test containers. The mixture in the containers should be allowed to solidify at room temperature for 20 - 30 minutes. If accumulation of humidity happens, the containers should be dried under aseptic conditions before proceeding to the inoculation.

If the cellulose and hemi cellulose mixture is used, it should be sterilized firstly (with humid vapor at 121 °C for 15 minutes or with ethylene oxide or by means of irradiation). Later it is placed in the final test containers and from 2 to 4 mL volumes of the previously prepared solution (per each container) are added.

In both of cases other layers formed by nitrocellulose or/and by other cellulose derivatives, such as cellulose acetate can be placed as well during as after inoculation, to provide the solid surface for developing of target organism colonies or to reveal a specific reaction.

The test samples can be inoculated by different streaking or dilution inoculation methods, and incubated at 33 to 45 °C, for at least 6 hours for the detection of *E. coli,* preferably between 12 and 22 hours and between 18 and 22 hours for the differentiation of other Gram-negative organisms.

The interpretation of the results is carried out by observing the color of the isolated colony and of the medium, presence of the opaque precipitate or colored zone around colonies (halo), the presence and color of fluorescent emissions and it is possible to observe the colonial morphology too.

The isolated colonies of different Gram-negative bacteria are observed as follows in table 1.

**Table 1. Characteristics of the colonies and of the medium.**

| **Microorganism** | **Colony color** | **Colony border** | **Color of medium** | **Precipitate or colored zone** | **Fluorescence** |
|---|---|---|---|---|---|
| *Escherichia coli,* except verotoxigenic strains | light violet | round | red | - | + blue |
| *Escherichia coli* O157:H7 | greenish blue | round | orange | - | - |
| *Shigella sonnei* | greenish blue | irregular | orange | - | + blue |
| *Shigella flexneri* | light rose | round | orange | - | blue in occasions |
| *Enterobacter* spp. | red with green greyish center | round | red | - | + yellow |
| *Citrobacter freundii* | dark violet | round | red | blue halo | - |
| *Klebsiella pneumoniae* | light violet | round | red | - | - |
| *Salmonella typhi* | red | round | red | Opaque precipitate | - |
| *Salmonella non-typhi* | red (with yellow center) | round | red | - | (+ yellow) |
| *Pseudomonas aeruginosa* | Light rose to (greenish brown) | irregular | orange | (greenish halo) | + greenish |
| *Proteus, Providencia* and other Gram-negative organisms | colorless | - | orange | - | - |

| | | | | | |
|---|---|---|---|---|---|
| ( ) Characteristics that can be observed after 24 hours of incubation | | | | | |

The different strains of Gram-negative bacteria develop colonies that can reach diameter of 5 mm, according to the incubation time.

### EXAMPLES

### Example No. 1

The composition of the nutrient mixture for the differentiation of Gram-negative bacterial strains was the following (table 2)

**Table 2. Composition of the mixture.**

| **INGREDIENT** | **% of dry weight** |
|---|---|
| Protein composition obtained by extraction of yeast cell content with a tryptophan contents from 0,6 to 0,8 % | 6,8 |
| Animal protein digest with a high content of the casein and a tryptophan from 0,8 to 1,2 % | 6,8 |
| Composition free of proteins and rich in animal origin protein fractions with tryptophan content from 0,5 to 0,7 % | 8,6 |
| Tryptophan | 2,5 |
| Sodium chloride | 9,2 |
| Sodium carbonate | 0,2 |
| Monopotassium phosphate | 3,1 |
| Dipotassium phosphate | 7,5 |
| Sodium piruvate | 1,0 |
| Sodium desoxycholate | 2,1 |
| Sorbitol | 20,7 |
| MUG | 0,1 |
| X-GAL | 0,1 |
| Neutral red | 0,06 |
| Agarose and agaropectin mixture | 31,24 |

| | |
|---|---|
| pH 7.0 ± 0.2 | |

48 g of this mixture was suspended in 1 L of deionized or distilled water.

The assay was carried out checking the behavior of different strains of Enterobacteria regarding the predetermined biochemical tests.

The obtained results (table 3) were the following:

**Table 3. Results of the growth in the mixture.**

| **Microorganism** | **Color (Medium)** | **Color (Colony)** | **CFU/mL (dil. 10⁻⁶)** | **Fluoresc.** | **Colony characteristics** |
|---|---|---|---|---|---|
| *E. coli* O157:H7 ATCC 35150 | pink | blue greenish | streaking | - | regular convex |
| *E. coli* ATCC 25922 | pink | violet with blue halo | 344 ± 60,8 | + blue | regular convex |
| *P. mirabilis* ATCC 12473 | pink | colorless to pale rose | 205 ± 13,4 | - | regular mucous |
| *Sh. sonnei* ATCC 25931 | orange | blue greenish | 367 ± 17,0 | + blue | irregular plane |
| *Sh. flexneri* ATCC 12022 | orange | pale rose | 136 ± 29,7 | - | regular convex |
| *A. hiodrofila* | orange | blue greenish | 255 ± 4,2 | - | regular plane |
| *S. typhimurium* ATCC 14028 | pink | Red | 324 ± 19,8 | - | regular convex |
| *P. vulgaris* ATCC 13315 | pink | colorless to rose | 272 ± 17,7 | - | regular convex |
| *Ps. aeruginosa* ATCC 27853 | pink | Pink | 130 ± 4,2 | + (green) | irregular convex |

After incubating for 24 hours at 35 ± 2 °C, a thin layer of cellulose impregnated with the Kovacs reagent, was placed in the container's lids. In the containers where indol-producing microorganisms were inoculated (*E. coli, P .vulgaris and Aeromonas hydrophila*), the color of the cellulose layer turned off the pink color in an interval of 30 min.

### Example No. 2

The nutrient mixture was prepared according to the composition described in the Example 1, but the content of the components of the group of tryptophan rich protein fractions was the following (table 4):

**Table 4. Composition of the mixture described in example 2.**

| **INGREDIENT** | **% of dry weight** |
|---|---|
| Protein composition obtained by yeast extraction with a tryptophan contents from 0,6 to 0,8 % | 11,54 |
| Animal protein digest with a high content of casein and tryptophan from 0,8 to 1,2 % | 11,54 |
| Composition free of proteins and rich in animal origin protein fractions with a tryptophan contents from 0,5 to 0,7 % | 4,62 |

The inoculation of different organisms from ATCC collection was made by the method of streaking the surface to obtain isolated colonies.

The interpretation of the results was carried out at 6, 11, 19 and 24 hours of incubation and the results are shown following (tables 5, 6, 7 and 8, respectively):

**Table 5. Growth at.6 hours of incubation.**

| **Microorganism** | **Observations** |
|---|---|
| *E. aerogenes* ATCC 13048 | The growth is observed in the first quadrant, the color of the mass of the colonies is pink |
| *E. coli* ATCC 25922 | The growth is observed in the first quadrant, the color of the mass of the colonies is violet. The blue fluorescence is observed in the place of growth. |
| *KI. pneumoniae* ATCC 13883 | Growth is not observed |
| *S. typhimurium* ATCC 14028 | Growth is not observed |
| *S. typhi* ATCC 19430 | Growth is not observed |

**Table 6. Growth at 11 hours of incubation.**

| **Microorganism** | **Observations** |
|---|---|
| *E. aerogenes* ATCC 13048 | The growth is observed in all the quadrants, the color of the colonies is red |
| *E. coli* ATCC 25922 | The growth is observed in several segments, the color of the mass of the colonies is red with blue spots. A more intense fluorescence is observed than for 6 h. of incubation |
| *Kl. pneumoniae* ATCC 13883 | A poor growth is observed. The color of the colonies is violet. |
| *S. typhimurium* ATCC 14028 | The colonies of red color are observed |
| *S. typhi* ATCC 19430 | The growth is observed in the first quadrant, the color of the colonies is red |

**Table 7. Growth at 19 hours of incubation.**

| **Microorganism** | **Observations** |
|---|---|
| *E. aerogenes* ATCC 13048 | The isolated colonies are red with the green center |
| *E. coli* ATCC 25922 | The colonies are of light violet color with blue florescence |
| *KI. pneumoniae* ATCC 13883 | The colonies are of violet color |
| *S. typhimurium* ATCC 14028 | The isolated colonies are red |
| *S. typhi* ATCC 19430 | The colonies are red with the opaque precipitate |

**Table 8. Growth at 24 hours of incubation.**

| **Microorganism** | **Observation** |
|---|---|
| *E. aerogenes* ATCC 13048 | Green points in the center of the colonies. Color more intense than at the 19 h. |
| *E. coli* ATCC 25922 | Isolated colonies are light violet. |
| *KI. pneumoniae* ATCC 13883 | The colonies maintain the violet color. |
| *S. typhimurium* ATCC 14028 | The isolated colonies are red. |
| *S. typhi* ATCC 19430 | The colonies are red with an opaque precipitate. |

It was paid attention to the fact that in the medium appears an opaque precipitate formation around of *S. typhi* colonies, which offers an additional characteristic for the differentiation of this specie from other *Salmonella* strains.

Subsequently, the same formulation of the nutrient mixture (following identified as **exp**) was inoculated with the 10⁻⁶ dilutions of the selected test organism suspensions (standardized to 50% transmittance).

The microorganisms were concurrently assayed in the EC Broth with MUG, adding agar (13 g/L) and X-GAL (0,1 g/L), and it was named as the reference mixture (**C**).

The observation of the growth results was carried out after 6, 12, 19, 24 and 40 hours of incubation (tables 9, 10 and 11). The results are shown as follows. Table 9. Growth at 6 hours of incubation.

| **Microorganism** | **Observations** |
|---|---|
| *E. aerogenes* ATCC 13048 | Growth is not observed |
| *E. coli* ATCC 25922 | Growth is not observed |
| *Kl. pneumoniae* ATCC 13883 | Growth is not observed |
| *E. cloacae ATCC 23355* | Growth is not observed |
| *S. typhimurium* ATCC 14028 | Growth is not observed |
| *S. typhi* ATCC 19430 | Growth is not observed |

**Table 10. Growth at 12 hours of incubation.**

| **Microorganism** | **Var** | **Color (Medium)** | **Color (Colony)** | **CFU** |
|---|---|---|---|---|
| *Escherichia coli* ATCC 25922 | exp | Growth is not observed | | |
| | C | | | |
| *Enterobacter aerogenes* ATCC 13048 | exp | red | red | 95 |
| | C | yellow | blue | 22 |
| *Enterobacter cloacae* ATCC 23355 | exp | red | red | 9 |
| | C | yellow | blue | 19 |
| *Klebsiella pneumoniae* ATCC 13883 | exp | Growth is not observed | | |
| | C | | | |
| *Salmonella typhimurium ATCC 14028* | exp | red | red | 11 |
| | C | yellow | colorless | 1 |
| *Salmonella typhi ATCC* 19430 | exp | Growth is not observed | | |
| | C | | | |

| | | | | |
|---|---|---|---|---|
| exp. : experimental C: control | | | | |

**Table 11. Growth at 19 hours of incubation .**

| **Microorganism** | **Var** | **Color of medium** | **Colony color** | **CFU** |
|---|---|---|---|---|
| *Escherichia coli* ATCC 25922 | exp | red | violet | 5 ± 3,5 |
| | C | yellow | blue | 30 ± 2,5 |
| *Enterobacter aerogenes* ATCC 13048 | exp | red | red with green center | 20 ± 16,3 |
| | C | yellow | blue | 26 ± 2,1 |
| *Enterobacter cloacae* ATCC 23355 | exp | red | red with grayish center | 20 ± 4,9 |
| | C | yellow | blue | 21 ± 4,2 |
| *Klebsiella pneumoniae* ATCC 13883 | Exp | red | violet | 11 ± 0,3 |
| | C | yellow | blue | 6 ± 2,8 |
| *Salmonella typhimurium ATCC 14028* | exp | red | red | 56 ± 9,2 |
| | C | yellow | colorless | 4 ± 0,7 |
| *Salmonella typhi* ATCC 19430 | exp | red | red | 34 ± 33,9 |
| | C | yellow | colorless | 25 ± 17,6 |

| | | | | |
|---|---|---|---|---|
| c. : center exp. : experimental C: control | | | | |

Surprisingly, in a different manner than for other coliform organisms, *E. aerogenes* and *E. cloacae* show a differentiated color in the center and in the borders of the colonies.

### Growth at 24 hours of incubation.

The colony numbers had not changed and the blue color in the colony centers became more intense.

### Growth at 40 hours of incubation.

The red color disappeared, and the blue color predominates in coliforms and the red *Salmonella* colonies were turned yellow.

### Example No. 3

It was carried out the study of the influence of different coliform organism growth activators, inhibitors and different combinations of tryptophan rich protein fractions by growth curve analysis of two strains:
*Escherichia coli* ATCC 10536
*Streptococcus faecalis* ATCC 29212
Concurrently was inoculated the EC Broth with MUG (Difco).

The experimental compositions (MN) taken as different experimental factors are shown in table 12.

**Table 12: Composition according the example 3.**

| **Ingredient** | **% of dry weight** | | | | |
|---|---|---|---|---|---|
| | **MN1** | **MN2** | **MN3** | **MN4** | **MN5** |
| Protein composition obtained by yeast extraction | 18,5 | 17 | 12,5 | 17,3 | 11 |
| Animal protein digest with a high content of casein | 18.5 | 17 | 12.5 | 17,3 | 16,5 |
| Composition free of proteins and rich in protein fractions of animal origin | 7,4 | 15 | 17,8 | 6,9 | 16,5 |
| Sodium desoxycholate | 3,6 | 3,3 | 0 | 3,5 | 0 |
| Bile salts | 0 | 0 | 4,5 | 0 | 4,4 |
| Sodium piruvate | 0 | 1,7 | 0 | 0 | 0 |
| Ammonium sulfate | 0 | 0 | 0 | 3,5 | 0 |
| Sorbitol | 35 | 29 | 35 | 34,5 | 34,4 |
| Sodium chloride | 17 | 17 | 17,7 | 17 | 17,2 |

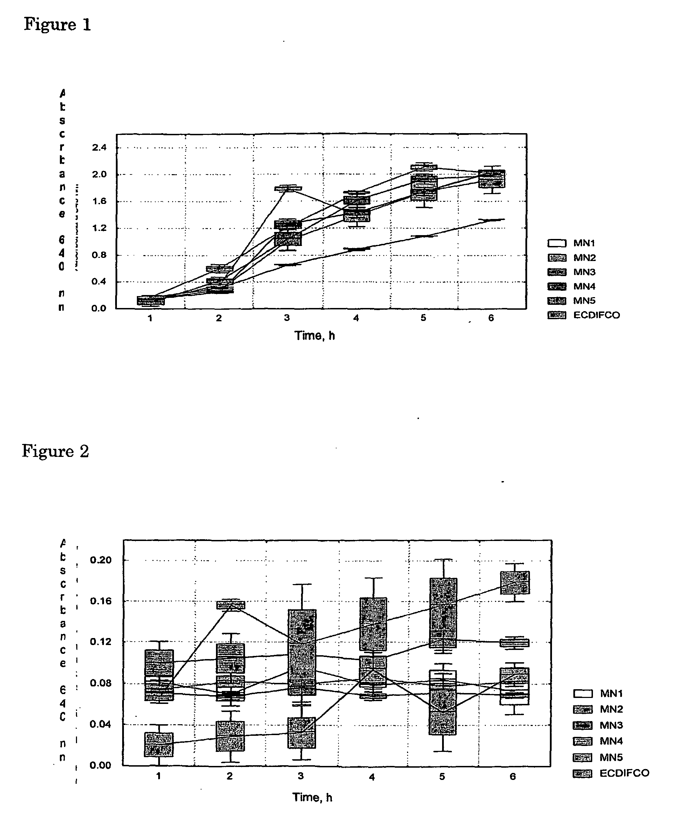

The obtained results are shown in Figures 1 and 2 of the annexes.

As it is shown in Figure 1, only in the MN3 nutrient mixture the growth rate of *E. coli* is smaller than in the reference mixes, while the MN2 variant promotes better the growth at 3 hours of incubation. At the 6 hours, practically there are no differences in the growth promotion between the variants, except the MN3 mixture.

In the Figure 2 it is shown that in all the compositions proposed in the present invention *Streptococcus faecalis* strain is inhibited better than in the reference diagnostic medium.

### Example No. 4

In this study was used a formulation shown in table 13.

**Table 13. Mixture according to example 4.**

| **INGREDIENT** | **% dry weight** |
|---|---|
| Protein composition obtained by extraction from yeasts, with a tryptophan content from 0,6 to 0,8 % | 8,3 |
| Digest of animal proteins with a high content of casein and a content of tryptophan from 0,8 to 1,2 % | 16,62 |
| Composition free of proteins and rich in protein fractions from animal origin with a tryptophan content from 0,5 to 0,7 % | 16,62 |
| Sodium chloride | 10,4 |
| Sodium carbonate | 0,1 |
| Sodium pyruvate | 2,1 |
| Bile salts | 2,7 |
| Sorbitol | 15,6 |
| MUG | 0,2 |
| X-GAL | 0,3 |
| Neutral red | 0,06 |
| Mixture of agarose and agaropectin | 27 |

| | |
|---|---|
| pH 7,0 ± 0,2 | |

In this study, the spread plating method was used for the inoculation.

All containers were incubated at 35 °C for 24 hours.

10⁻⁶ diluted suspensions of coliform microorganism, *Escherichia coli* ATCC 25922 and *Enterobacter aerogenes* ATCC 13048, and the 10⁻¹ diluted suspension of *Streptococcus faecalis* ATCC 29212 were inoculated.

The EC Broth with MUG (Difco) with addition of agar (13,0 g/L), was used as a reference medium and was denominated as "control"(C).

There were achieved the following results (table 14):

**Table 14. Results of the growth of microorganisms in experimental and control mixtures.**

| **Microorganism** | **Var** | **Color (Medium)** | **Color (Colony)** | **Fluoresc.** | **CFU/mL** |
|---|---|---|---|---|---|
| *Escherichia coli* ATCC 25922 (10⁻⁵ dil.) | exp | red | red with blue center | + | 361 ± 23,4 |
| | C | yellow | white | + | 313 ± 44,6 |
| *Enterobacter aerogenes* ATCC 13048 (10⁻⁵ dil.) | exp | red | red with green center | - | 284 ± 33,4 |
| | C | yellow | white | - | 137 ± 18,3 |

| | | | | | |
|---|---|---|---|---|---|
| exp: mixture described in the present invention C: control formulation | | | | | |

There is no significant difference (p<0,05) in *Escherichia coli* ATCC 25922 CFU counts between the experimental variant and the EC Broth with MUG (Difco), although the size of the colonies in the experimental nutrient mixture is lightly bigger. The number of *Enterobacter aerogenes* ATCC 13048 colonies is higher in the experimental variant; nevertheless there are no significant differences among of them (p ≅0,06).

Surprisingly, *E. aerogenes* presented a differed coloration in the center and in the borders of the colony and it could be identified by its yellow fluorescence.

On the other hand, *Streptococcus faecalis* ATCC 29212 was inhibited completely in all the studied variants (the microorganism was incubated up to 72 hours).

Subsequently 10 mL of potable water sample from a storage tank was inoculated in the same nutrient mixture variants (table 15).

**Table 15. Detection of the growth in the sample of potable water.**

| | | | | | |
|---|---|---|---|---|---|
| **Microorganism** | **Var** | **Color (Medium)** | **Color (Colony)** | **Fluoresc.** | **CFU/mL** |
| Basin potable water | exp | red | red with greenish center | - | 44 ± 4,6 |
| | C | yellow | green greyish | - | 16 ± 4,2 |

| | | | | | |
|---|---|---|---|---|---|
| exp: mixture described in the present invention C: control formulation | | | | | |

It was carried out only the count of the colonies with the presumptive coliform characteristics. By the carried out biochemical tests (next shown) was confirmed that the isolated colonies belonged to the *Enterobacter* genus.

| **Test** | **Result** |
|---|---|
| Citrate utilization | + |
| Lysine decarboxylation | + |
| Ornitine decarboxylation | + |
| Arginine decarboxylation | - |
| Kligler Iron Agar | A/A Gas + H₂S- |
| Urease | - |
| Motility | + |
| Indol production | - |
| Methyl red | - |
| Voges-Proskauer | + |

There is significant difference (p < 0,05) in colony counts among the experimental variant of the nutrient mixture and the reference medium (Difco) with the addition of agar (13g/L) and X-Gal (1,0 g/L), in favor of the first.

### Example No. 5

It was prepared the same nutrient mixture as in the Example No. 4, but the total quantity of the tryptophan rich protein fractions was established in: 40,4; 37,7 y 34,8 % of dry weight (V1, V2 and V3, respectively).

The three variants were assayed with and without the agarose and agaropectin mixture. In the first three variants the 10⁻⁶ dilutions of *Escherichia coli* ATCC 25922 and *Salmonella typhimurium* ATCC 14028 were inoculated by spreading plate method.

In this study the following results were obtained (table 16):

**Table 16. Growth of different organisms in the experimental mixture .**

| **Microorganism** | **Var** | **Color of medium** | **Colony color** | **CFU/mL** |
|---|---|---|---|---|
| *Escherichia coli* ATCC 25922 (10⁻⁵ dilution) | V1 | red | red with blue center | 20 ± 4,6 |
| | V2 | red | red with blue center | 22 ± 4,0 |
| | V3 | red | red with blue center | 15 ± 2,6 |
| *Salmonella typhimurium* ATCC 14028 (10⁻⁵ dilution) | V1 | red | Red | 46 ± 13,0 |
| | V2 | red | Red | 47 ± 8,7 |
| | V3 | red | Red | 36 ± 15,0 |

The colony counts of *Escherichia coli* ATCC 25922, *Enterobacter aerogenes* ATCC 13048 and *Salmonella typhimurium* ATCC 14028 performed on all of the variants were similar (p>0,05).

Concurrently, the other three variants without the mixture of agarosa and agaropectina were used for the growth curve analysis of Gram-negative (positive control) and Gram-positive microorganisms (negative control). The reference media used was EC Broth with MUG from Difco.

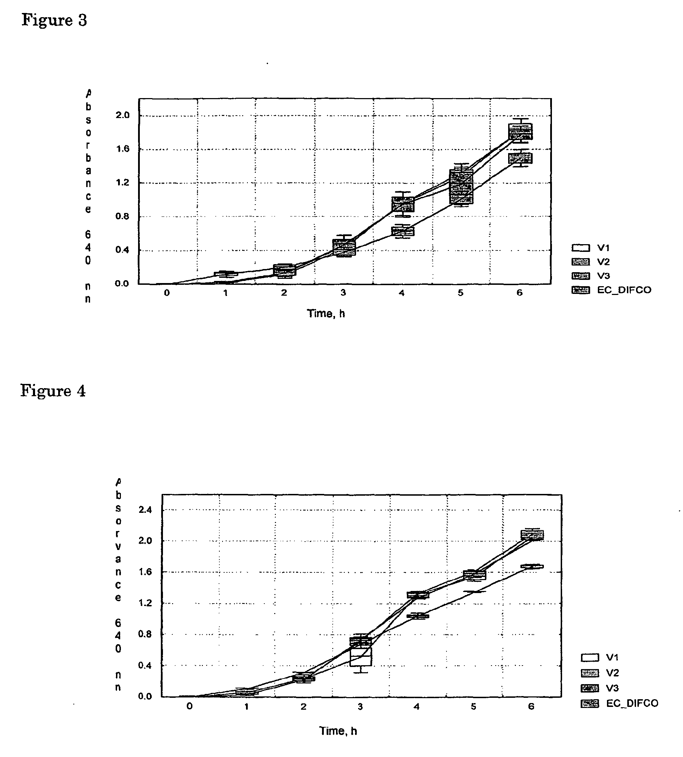

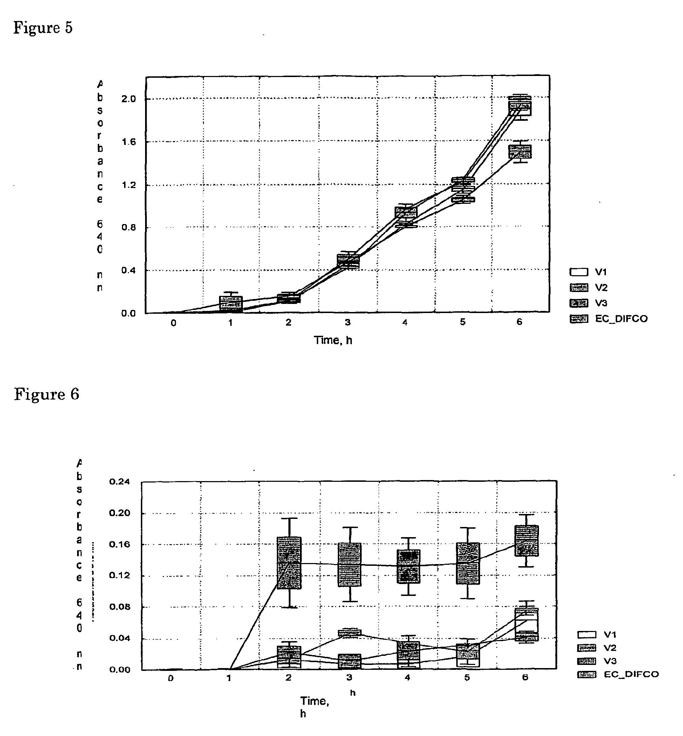

Surprisingly it was observed that at 6 h of incubation, the growth rate (absorbance) of *Escherichia coli* ATCC 25922, *Enterobacter aerogenes* ATCC 13048 and *Salmonella typhimurium* ATCC 14028 strains in the three experimental variants was significantly higher (p <0,05) than in the reference medium. While the inhibition of *Streptococcus faecalis* ATCC 29212 strain (negative control) was significantly higher in the same variants corresponding to the present invention (Figures 3-6).

### Example No. 6

It was prepared the nutrient mixture as the V2 variant of the example No. 5, with double straight concentrations of neutral red and of MUG. The agarose and agaropectin mixture was substituted by the mixture of cellulose and hemi cellulose to provide a solid structure for the development of the target organism's colonies (exp). All above-mentioned ingredients form the first layer of the nutrient mixture.

It was inoculated by membrane filtration method 1 mL of 10⁻⁶ diluted suspensions of the microorganisms; representatives of several genera of Enterobacteria, of 10⁻⁵ diluted *Klebsiella pneumoniae* ATCC 13883 and 10⁻¹ diluted *Staphylococcus aureus* ATCC 25923 (negative control) suspensions. To filter was used nitrate cellulose support, which was placed as another layer of the nutritious mixture.

Concurrently the Violet Red Bile Agar was inoculated by spreading plate method (C).

The results of the examination at 18 h of incubation are shown in table 17:

**Table 17. Growth of different strains in the mixture described in example 6.**

| **Microorganism** | **Var** | **Color (Medium)** | **Color (Colony)** | **Fluoresc.** | **CFU** |
|---|---|---|---|---|---|
| *Escherichia coli* ATCC 25922 | exp | red | light violet | + blue | 170 ± 7,8 |
| | C | red | red | - | 167 ± 23,3 |
| *Escherichia coli 0157 H:7* ATCC 35150 | exp | orange | blue greenish | - | 192 ± 11,5 |
| | C | red | red | - | 103 ± 18,7 |
| *Enterobacter aerogenes* ATCC13048 | exp | red | red with green center | + yellow | 272 ± 37,5 |
| | C | red | red | - | 99 ± 76,8 |
| *Salmonella typhimurium* ATCC 14028 | exp | red | red | - | 26 ± 11,1 |
| | C | without change | colorless | - | 24 ± 23,6 |
| *Citrobacter freundii* ATCC 8090 | exp | red | dark violet with blue halo | - | 205 ± 6,43 |
| | C | red | red | - | 166 ± 13,7 |
| *Klebsiella pneumoniae* ATCC 13883 | exp | red | dark violet | - | 115 ± 40,3 |
| | C | red | red | - | 277 ± 98.5 |
| *Shigella sonnei* ATCC 25931 | exp | orange | blue greenish | +(blue) | 159 ± 28,6 |
| | C | without change | colorless | - | 92 ± 13.6 |
| *Shigella flexneri* ATCC 12022 | exp | orange | colorless or pale rose | - | 181 ± 52,9 |
| | C | without change | colorless | - | 9 ± 1,5 |

| | | | | | |
|---|---|---|---|---|---|
| exp: mixture described in the present invention C: control formulation | | | | | |

### Escherichia coli, Salmonella typhimurium, Klebsiella pneumoniae, Citrobacter freundii,

*Shigella sonnei* and *Enterobacter aerogenes* colony counts in the experimental nutrient mixture and the Violet Red Bile Agar were not significantly different (p < 0.05). While *Escherichia coli* O157:H7 and *Shigella flexneri* colony counts were significantly higher in the nutrient mixture. The low diluted (10⁻¹) suspension of *Staphylococcus aureus* was completely inhibited (incubation up to 48 h).

A comparative study of recovery of different Gram-negative bacteria in the same composition of the nutrient mixture and in m- Endo broth (Difco) was carried out. Both media were inoculated by membrane filtration method, obtaining the following results (table 18):

**Table 18. Growth of E. coli and E. coli 0157:H7.**

| **Microorganism** | **Var** | **Color (Medium)** | **Color (Colony)** | **Fluoresc.** | **UFC** |
|---|---|---|---|---|---|
| *Escherichia coli* ATCC 25922 | exp | red | light violet | + blue | 185 ± 10,7 |
| | Endo | red | dark red with greenish metallic sheen | - | 170 ± 7,8 |
| *Escherichia coli 0157 H:7* ATCC 35150 | exp | orange | greenish blue | - | 158 ± 8,5 |
| | Endo | red | dark red with greenish metallic sheen | - | 192 ± 11,5 |
| *Enterobacter aerogenes* ATCC 13048 | exp | red | red with green center | + yellow | 215 ± 47,1 |
| | Endo | red | red with light metallic sheen | - | 272 ± 37,5 |
| *Salmonella typhimurium* ATCC 14028 | exp | red | red | - | 17,7 ± 6,4 |
| | Endo | red | red | - | 26 ± 11,1 |
| *Citrobacter freundii* ATCC 8090 | exp | red | dark violet with blue halo | - | 156 ± 48,8 |
| | Endo | red | red with dark center | - | 205 ± 6,43 |
| *Klebsiella pneumoniae* ATCC 13883 | exp | red | dark violet | - | 114 ± 88,9 |
| | Endo | red | dark red | - | 115 ± 40,3 |

The differentiation of different representatives of the coliform group is evident in the nutrient mixture. However, in m-Endo broth *Enterobacter aerogenes* colonies can be easily mistaken with some *E. coli* colonies, which possess a light metallic sheen.

Unexpectedly a blue halo (colony surrounding zone) formation was detected around the colonies of *Citrobacter freundii* in this composition of the nutrient mixture.

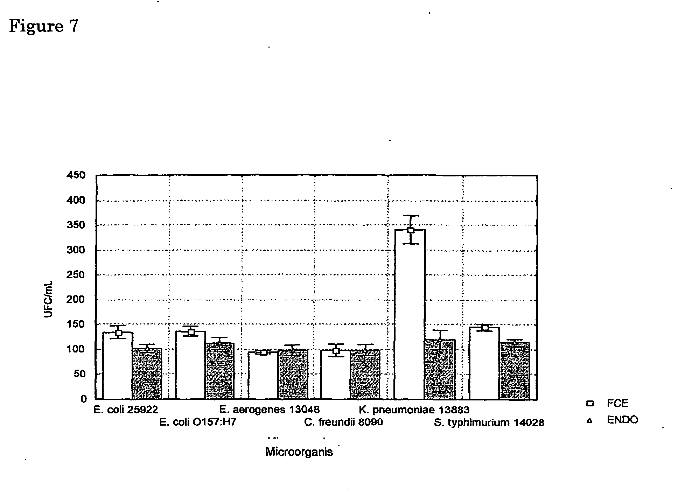

*E. coli* ATCC 25922, *K. pneumoniae* ATCC 13883 and *S. typhimurim* ATCC 14028 colony counts in the experimental mixture were significantly higher (p< 0.05) while the colony numbers of other microorganisms on both mixtures were similar. These results are shown in the Fig. 7 of the annexes.

### Example No. 7

The nutrient mixture with the same composition of the variant V2 of the Example No. 5 and S.S. Agar were inoculated at once.

The objective of this study was to compare the specificity of the nutrient mixture, subject of the present invention, and S.S Agar for the detection of *Salmonella* from a polymicrobial mixture.

The inoculum was prepared as follows:

The primary suspensions of *Salmonella typhimurium* ATCC 14028, *Citrobacter freundii* ATCC 8090, *Serratia marcescens* ATCC 8100, *Enterobacter cloacae* ATCC 23355 and *Pseudomonas aeruginosa* ATCC 27835 were prepared from one freshly grown colony in 10 mL of sterile saline solution.

Decimal dilutions (up to 10⁻⁴) were prepared from each suspension. Then 1 mL of each 10⁻⁴ dilution was added to a tube with 9 mL of sterile saline solution and mixed thoroughly on a vortex mixer.

Enrichment was performed in 10 mL of Selenite Broth inoculated with 1 mL of this mixture, which was incubated for 24 hours at 43 °C.

A sample taken with a calibrated loop from the Selenite Broth culture was inoculated onto Petri dishes with the nutrient mixture, according to the invention, and S.S Agar. All plates were incubated at 43 °C.

The growth results were inspected after incubation for 24 hours. Three types of colonies were observed in the plates with the nutrient mixture:
1. Blue colonies with a blue halo (*Citrobacter freundii*, presumably)
2. Red colonies with a yellowish center (*Salmonella*, presumably)
3. Colorless colonies with a rose center and greenish fluorescence (*Pseudomonas aeruginosa,* presumably).

In S.S Agar four types of colonies were observed:
1. Big white colonies with a black center (*Salmonella* or *Citrobacter*)
2. Medium colorless colonies with a black center (*Salmonella* or *Citrobacter*)
3. Big rose colonies (Coliforms)
4. Small rose colonies (Coliforms).

The biochemical tests yielded the following results (table 19):

**Table 19. Biochemical confirmatory tests.**

| **Test** | **Mixture proposed in the invention** | | | **S.S agar** | | | |
|---|---|---|---|---|---|---|---|
| | 1 | 2 | 3 | 1 | 2 | 3 | 4 |
| Citrate utilization | + | + | + | + | + | + | + |
| Sorbitol fermentation | + | + | - | + | + | + | - |
| Lysine decarboxylation | - | + | + | - | + | + | + |
| Kligler iron agar | A/A++ | K/A++ | K/K-- | K/A++ | K/A++ | K/A+- | K/K-- |
| Urea utilization | - | - | - | - | - | + | - |
| Ramnose fermentation | + | + | - | + | + | + | - |
| H₂S production | + | + | - | + | - | - | - |
| Indol production | - | - | - | - | - | - | - |
| Motility | + | + | + | + | + | - | + |

The results of the presumptive identification in the nutrient mixture object of the invention, had been confirmed by serological tests with *Salmonella* polyvalent antiserum.

In S.S Agar were confirmed as *Salmonella* only the colorless colonies (they are lysine decarboxylase +).

### Example 8.

The nutritive mixture was prepared according to the composition described in table 20. Table 20. Composition of the mixture according to the example 8.

| **INGREDIENT** | **% dry weight** |
|---|---|
| Mixture of protein fractions rich in tryptophan | 36,85 |
| Sodium chloride | 16 |
| Sodium carbonate | 0,33 |
| Bile salts | 4,2 |
| MUG | 0,16 |
| X-GAL | 0,16 |
| Mixture of agarose and agaropectin | 42,3 |

The functionality of the nutritive mixture was evaluated for the diagnosis of *E. coli and* coliforms in urine samples (where infection by *E. coli* and coliforms are about 90 % of the urinary sepsis). Later, a confirmation of the pathogens, which originated the urinary sepsis, was executed by biochemical tests.

In the next table 21, are summarized the values of the incidence of all the identified pathogens.

**Table 21. Incidence of the pathogens, identified by biochemical tests.**

| Microorganism | No. of positive samples | Incidence, % |
|---|---|---|
| *Escherichia coli* | 154 | 72,6 |
| *Citrobacter sp.* | 9 | 4,2 |
| *Proteus sp.* | 16 | 7,6 |
| *Pseudomonas sp* | 4 | 1,9 |
| *Providencia sp* | 1 | 0,5 |
| *Klebsiella sp.* | 12 | 5,7 |
| *Serratia sp.* | 3 | 1,4 |
| *Gram positives* | 5 | 2,4 |
| *Acinetobacter* | 2 | 0,9 |
| *Enterobacter sp* | 6 | 2,8 |
| Total | 212 | 100 |

The diagnostic sensibility and specificity were determined in the experimental mixture.

During the evaluation, no false negative were found after the identification, and this fact allow assuring that the diagnostic specificity was 100 %. It was observed fluorescence in one strain of *Klebsiella*, considering it as a false positive result. Due to this fact, the diagnostic sensitivity was 98,04 %.

### Brief Description of the Figures:

**Figure 1:** Results of the growth of *E. coli* ATCC 10536. It is observed that only in the MN3 nutrient mixture the growth rate of *E. coli* is smaller than in the reference mixes, while the MN2 variant promotes better the growth at 3 hours of incubation. At the 6 hours, practically there are no differences in the growth promotion between the variants, except the MN3 mixture.
**Figure 2:** Growth of *Streptococcus faecalis* ATCC. Ti is observed that in all the proposed inventive mixtures, *Streptococcus faecalis* was inhibited in comparison with the reference diagnostic medium.
**Figure 3:** Growth of *Escherichia coli* ATCC 25922 in compositions V1, V2 and V3 and in the reference medium.
**Figure 4:** Growth of *Enterobacter aerogenes* ATCC 13048 in the mixtures V1, V2 and V3 and in the reference medium.
**Figure 5:** Growth of *Salmonella typhimurium* ATCC 14028 in the mixtures V1, V2 and V3 and in the reference medium.
**Figure 6:** Growth of *Streptococcus faecalis* ATCC 29212 in mixture V1, V2 and V3 and in the reference medium.
**Figure 7:** Behavior of different microorganisms in the experimental mixture (FCE) and in the reference composition.

## Claims

1. A nutrient mixture for the identification and early counting of Gram-negative organisms wherein said mixture contains:
- a mixture of tryptophan rich protein fractions or free tryptophan in quantities from 22 to 46 % of the total mixture (weight/weight), and wherein said amino acid tryptophan is in quantities from 0,25 to 3,8 % of said mixture;
- a mixture of organic and/or inorganic salts in quantities from 15 to 20 % of the total mixture (weight/weight) wherein said organic and/or inorganic are selected from the group consisting of NaCl, K₂HPO₄, KH₂PO₄, (NH₄)₂SO₄, Na₂CO₃. and sodium piruvate and their mixtures, preferably been selected NaCl and Na₂CO₃. which are in said mixture in the following amounts:
NaCl from 7 to 18 %;
K₂HPO₄ from 6 to 11 %;
KH₂PO4 from 2 to %;
(NH₄)₂SO₄ from 1 to 4 %;
Na₂CO₃ from 0,1 to 0,4 %; and
Sodium Piruvate from 0,7 to 3 %.
- a mixture of substances providing color or fluorescence in quantities from 0,3 to 37 % of the total mixture (weight/weight) wherein said substances are selected from the group consisting of X-GAL, MUG, Sorbitol and Neutral Red, been selected preferably X-GAL y MUG, and they are in said mixture in the.following amounts:
X-GAL from 0,16 to 0,36 %;
MUG from 0,16 to 0.18%;
Sorbitol from 15 to 36,5 %; and
Neutral Red from 0,09 to 0,11 %.;
- inhibitor substances of the Gram-positive organisms in quantities from 2 to 4,5 % of the total mixture (weight/weight); and
- a mixture of substances providing solid structure to the medium, in quantities from 20 to 50 %;
having said nutrient mixture once prepared a pH from 6,6 to 7,2.

2. A nutrient mixture according to claim 1 wherein the inhibitor substances of the Gram-positive organisms are sodium desoxycholate and bile salts.

3. A nutrient mixture according to claim 1 wherein the mixture of substances providing solid structure to the medium comprises the following combinations and amounts (weight/weight) in respect to the total mixture:
- agarose and agaropectina in quantities from 19 to 48 %, with cellulose nitrate in quantities from 0,1 to 0,4 %; or
- cellulose and hemi cellulose in quantities from 1,4 to 3 %, with cellulose nitrate in quantities from 0,1 to 0,4 %, or
- agarose and agaropectina only, in quantities from 19 to 48 %.

4. A procedure for the identification and early count of Gram negative organisms, wherein the nutritive mixture of any of the claims from 1 to 3, after its solidification and being in contact with the microorganisms or samples containing them, it is incubated for a period from 12 to 22 hours, at a temperature from 30 to 45 °C, from which it is possible the identification of said microorganisms at first sight, while fluorescence detection is carried out under ultraviolet light from 360 to 366 nm.

5. A procedure according to claim 4 wherein the identification of the microorganisms is possible by the appearance of five different colors of the colonies, of fluorescent emissions of three colors, of halos of three colors, of color changes in the medium, and of the appearance of a precipitation zones surrounding the colonies, as well as the combinations of these characteristics.

6. A procedure according to claim 5, wherein the identification of the organisms to detect is carried out as follows:
*Shigella sonnei-* By the appearance of blue greenish colonies with blue fluorescence and the orange medium;
*Shigella flexneri -* By the appearance of pale rose colonies, yellowish halo and the medium color is orange;
*Enterobacter (E. aerogenes, E. cloacae, E. agglomerans) -* By the appearance of pink to red colonies with green greyish center, yellow fluorescence and the red medium;
*Escherichia coli*, except verotoxigenic strains - By the appearance of light violet colonies, blue fluorescence and red medium;
*Escherichia coli* O157:H7 - By the appearance of blue greenish colonies and orange medium;
*Citrobacter freundii* - By the appearance of dark violet colonies with blue halo and red medium;
*Klebsiella pneumoniae -* By the appearance of light violet colonies and red medium;
*Salmonella typhi* - By the appearance of red colonies and red medium with a zone of opaque precipitate;
*Sabnonella* "no typhi" - By the appearance of red colonies and red medium; after 24 h of incubation the color of the center becomes yellow and shows a yellow fluorescence;
*Pseudomonas aeruginosa* - By the appearance of pale rose colonies, greenish fluorescence before the 24 h of incubation and orange medium; greenish brown colonies and greenish surrounding zone, greenish fluorescence after the 24 h of incubation;
*Proteus, Providencia, Alcaligenes* and other Gram-negative organisms - By the appearance of colorless or transparent colonies and orange medium.

7. A procedure according to claim 6, wherein when using X-gal and MUG as sole components of a mixture that provide the color or fluorescence, the identification of total conforms is made by the blue greenish color of the colonies, and specifically for *E. coli,* also by its blue fluorescence.

## Patentansprüche

1. Nährmixtur für das Identifizieren und frühe Zählen von Gramnegativen Organismen, wobei die Mixtur enthält:
- eine Mixtur aus tryptophanreichen Proteinfraktionen oder freies Tryptophan in Mengen von 22 bis 46% (Gewicht/Gewicht) der gesamten Mixtur, wobei die Aminosäure Tryptophan in Mengen von 0,25 bis 3,8% der Mixtur vorhanden ist;
- eine Mixtur aus organischen und/oder anorganischen Salzen in Mengen von 15 bis 20% (Gewicht/Gewicht) der gesamten Mixtur, wobei die organischen und/oder anorganischen Salze ausgewählt sind aus der aus NaCl, K₂HPO₄, KH₂PO₄, (NH₄)₂SO₄, Na₂CO₃, und Natriumpyruvat und deren Mixturen bestehenden Gruppe, bevorzugt ausgewählt aus NaCl und Na₂CO₃, und in der Mixtur vorhanden sind in den folgenden Mengen:
NaCl von 7 bis 18%;
K₂HPO₄ von 6 bis 11 %;
KH₂PO₄ von 2 bis 5%;
(NH₄)₂SO₄ von 1 bis 4%;
Na₂CO₃ von 0,1 bis 0,4%; und
Natriumpyruvat von 0,7 bis 3%;
- eine Mixtur aus Substanzen, die Farbe oder Fluoreszenz zur Verfügung stellt, in Mengen von 0,3 bis 37% (Gewicht/Gewicht) der gesamten Mixtur, wobei die Substanzen ausgewählt sind aus der aus X-GAL, MUG, Sorbitol und Neutralrot bestehenden Gruppe, bevorzugt ausgewählt aus X-GAL und MUG, und in der Mixtur vorhanden sind in den folgenden Mengen:
X-GAL von 0,16 bis 0,36%;
MUG von 0,16 bis 0,18%;
Sorbitol von 15 bis 36,5%; und
Neutralrot von 0,09 bis 0,11 %;
- Inhibitorsubstanzen der Gram-positiven Organismen in Mengen von 2 bis 4,5% (Gewicht/Gewicht) der gesamten Mixtur; und
- eine Mixtur aus Substanzen, die dem Medium eine feste Struktur zur Verfügung stellt, in Mengen von 15 bis 50%;
wobei die einmal fertig gestellte Nährmixtur einen pH von 6,6 bis 7,2 aufweist.

2. Nährmixtur nach Anspruch 1, wobei
die Inhibitorsubstanzen der Gram-positiven Organismen Natriumdesoxycholat und Gallensalze sind.

3. Nährmixtur nach Anspruch 1, wobei
die Mixtur aus Substanzen, die dem Medium feste Struktur zur Verfügung stellt, die folgenden Kombinationen und Mengen (Gewicht/Gewicht) in Bezug auf die gesamte Mischung enthält:
- Agarose und Agaropectin in Mengen von 19 bis 48%, mit Cellulosenitrat in Mengen 0,1 bis 0,4%; oder
- Cellulose und Hemicellulose in Mengen von 1,4 bis 3%, mit Cellulosenitrat in Mengen von 0,1 bis 0,4%, oder
- nur Agarose und Agaropectin, in Mengen von 19 bis 48%.

4. Verfahren für das Identifizieren und frühe Zählen von Gramnegativen Organismen, wobei die Nährmixtur nach einem der Ansprüche 1 bis 3 nach ihrem Festwerden und nachdem sie mit den Mikroorganismen oder den sie enthaltenden Proben in Kontakt gebracht worden ist, für einen Zeitraum von 1 2 bis 22 Stunden bei einer Temperatur von 30 bis 45°C inkubiert wird, so dass das Identifizieren der Mikroorganismen auf den ersten Blick möglich ist, während Fluoreszenznachweis unter ultraviolettem Licht von 360 bis 366 nm ausgeführt wird.

5. Verfahren nach Anspruch 4, wobei
das Identifizieren der Mikroorganismen möglich ist durch das Auftreten von fünf verschiedenen Farben der Kolonien, von Fluoreszenzemissionen von drei Farben, von Halos von drei Farben, von Farbänderungen in dem Medium und vom Auftreten einer Niederschlagszone, die die Kolonien umgibt, sowie durch die Kombinationen dieser Charakteristika.

6. Verfahren nach Anspruch 5, wobei
das Identifizieren der nachzuweisenden Organismen wie folgt ausgeführt wird:
*Shigella sonnei -* Durch das Auftreten von blau-grünlichen Kolonien mit blauer Fluoreszenz und dem orangen Medium;
*Shigella flexneri -* Durch das Auftreten von blassrosa Kolonien, gelblichem Halo und oranger Farbe des Mediums;
*Enterobacter (E. aerogenes, E. cloacae, E. agglomerans) -* Durch das Auftreten von pinkfarbenen bis roten Kolonien mit grün-gräulichem Zentrum, gelber Fluoreszenz und dem roten Medium;
*Escherichia coli,* mit Ausnahme von verotoxigenen Stämmen - Durch das Auftreten von hellvioletten Kolonien, blauer Fluoreszenz und rotem Medium; *Escherichia coli* 0157:H7 - Durch das Auftreten von blau-grünlichen Kolonien und orangem Medium;
*Citrobacter freundii -* Durch das Auftreten von dunkelvioletten Kolonien mit blauem Halo und rotem Medium;
*Klebsiella pneumoniae -* Durch das Auftreten von hellvioletten Kolonien und rotem Medium;
*Salmonella typhi -* Durch das Auftreten von roten Kolonien und rotem Medium mit einer Zone opaken Niederschlags;
*Salmonella* "nicht typhi" - Durch das Auftreten von roten Kolonien und rotem Medium; nach 24 Stunden Inkubation wird die Farbe des Zentrums gelb und zeigt eine gelbe Fluoreszenz;
*Pseudomonas aeruginosa -* Durch das Auftreten von blassrosa Kolonien, grünlicher Fluoreszenz vor den 24 Stunden Inkubation und orangem Medium; grünlich-braune Kolonien und grünliche umgebende Zone, grünliche Fluoreszenz nach den 24 Stunden Inkubation;
*Proteus, Providencia, Alcaligenes* und andere Gram-negative Organismen - Durch das Auftreten von farblosen oder transparenten Kolonien und orangem Medium.

7. Verfahren nach Anspruch 6, wobei
bei Verwendung von X-GAL und MUG als einzige Komponenten einer Mixtur, die die Farbe oder Fluoreszenz zur Verfügung stellt, das Identifizieren von Gesamt-Coliformen durch die blau-grünliche Farbe der Kolonien, und insbesondere bei *E. coli* auch durch ihre blaue Fluoreszenz erfolgt.

## Revendications

1. Mélange de nutriments pour l'identification et le dénombrement rapide de germes Gram négatif, où ledit mélange contient:
- un mélange de fractions protéiques riches en tryptophane ou de tryptophane libre en des quantités allant de 22 à 46% du mélange total (poids/poids), et dans lequel ledit acide aminé tryptophane est présent en des quantités allant de 0,25 à 3,8% dudit mélange;
- un mélange de sels organiques et/ou inorganiques en des quantités allant de 15 à 20% du mélange total (poids/poids), où lesdits sels organiques et/ou inorganiques sont choisis dans le groupe constitué par NaCl, K₂HPO₄, KH₂PO₄, (NH₄)₂SO₄, Na₂CO₃, et le piruvate de sodium ainsi que leurs mélanges, de préférence choisis parmi NaCl et Na₂CO₃, qui sont présents dans ledit mélange aux quantités suivantes:
NaCl de 7 à 18%;
K₂HPO₄ de 6 à 11%;
KH₂PO₄ de 2 à 5%;
(NH₄)₂SO₄ de 1 à 4%;
Na₂CO₃ de 0,1 à 0,4%; et
Piruvate de sodium de 0,7 à 3%;
- un mélange de substances donnant une couleur ou une fluorescence en des quantités allant de 0,3 à 37% du mélange total (poids/poids), où lesdites substances sont choisies dans le groupe constitué par X-GAL, MUG, Sorbitol et Rouge Neutre, de préférence choisies parmi X-GAL et MUG, et elles sont présentes dans ledit mélange aux quantités suivantes:
X-GAL de 0,16 à 0,36%;
MUG de 0,16 à 0,18%;
Sorbitol de 15 à 36,5%, et
Rouge Neutre de 0,09 à 0,11 %;
- des substances inhibitrices des germes Gram positif en des quantités allant de 2 à 4,5% du mélange total (poids/poids); et
- un mélange de substances donnant une structure solide au milieu, en des quantités allant de 20 à 50%;
ledit mélange de nutriments, une fois préparé, ayant un pH de 6,6 à 7,2.

2. Mélange de nutriments selon la revendication 1, dans lequel les substances inhibitrices des germes Gram positif sont le désoxycholate de sodium et les sels biliaires.

3. Mélange de nutriments selon la revendication 1, dans lequel le mélange de substances donnant une structure solide au milieu comprend les combinaisons et les quantités (poids/poids) suivantes par rapport au mélange total:
- agarose et agaropectine en des quantités allant de 19 à 48%, avec du nitrate de cellulose en des quantités allant de 0,1 à 0,4%; ou
- cellulose et hémicellulose en des quantités allant de 1,4 à 3%, avec du nitrate de cellulose en des quantités allant de 0,1 à 0,4%, ou
- agarose et agaropectine seuls, en des quantités allant de 19 à 48%.

4. Mode opératoire pour l'indentification et le dénombrement rapide de germes Gram négatif, dans lequel le mélange nutritif selon l'une quelconque des revendications 1 à 3, après sa solidification et après mise en contact avec les microorganismes ou les échantillons les contenant, est incubé pendant une période de 12 à 22 heures, à une température de 30 à 45°C, d'où il est possible d'identifier lesdits microorganismes à première vue, tandis que la détection par fluorescence est réalisée sous une lumière ultraviolette de 360 à 366 nm.

5. Mode opératoire selon la revendication 4, dans lequel l'identification des microorganismes est possible grâce à l'apparition de cinq couleurs différentes des colonies, d'émissions fluorescentes de trois couleurs, d'auréoles de trois couleurs, de changements de couleur dans le milieu, et l'apparition d'une zone de précipitation entourant les colonies, ainsi que les combinaisons de ces caractéristiques.

6. Mode opératoire selon la revendication 5, dans lequel l'identification des germes à détecter s'effectue comme suit:
*Shigella sonnei* - Par l'apparition de colonies bleues verdâtres avec une fluorescence bleue et le milieu orange;
*Shigella flexneri* - Par l'apparition de colonies roses pâles, une auréole jaunâtre et le milieu est coloré en orange;
*Enterobacter (E. aerogenes, E. cloacae, E. agglomerans)* - Par l'apparition de colonies de couleur rose à rouge avec un centre vert grisâtre, une fluorescence jaune et un milieu rouge,
*Escherichia coli,* à l'exception des souches vérotoxigéniques - Par l'apparition de colonies violet clair, une fluorescence bleue et un milieu rouge;
*Escherichia coli* O157:H7 - Par l'apparition de colonies bleu verdâtre et un milieu orange;
*Citrobacter freundii -* Par l'apparition de colonies violet foncé avec une auréaole bleue et un milieu rouge;
*Klebsiella pneumoniae* - Par l'apparition de colonies violet clair et un milieu rouge;
*Salmonella typhi* - Par l'apparition de colonies rouges et un milieu rouge avec une zone de précipité opaque;
*Salmonella* "non typhi" - Par l'apparition de colonies rouges et un milieu rouge, après 24 h d'incubation, la couleur du centre vire au jaune et présente une fluorescence jaune;
*Pseudomonas aeruginosa -* Par l'apparition de colonies roses pâles, une fluorescence verdâtre avant les 24 h d'incubation et un milieu orange; des colonies brun verdâtre et une zone environnante verdâtre, une fluorescence verdâtre après les 24 h d'incubation;
*Proteus, Providencia, Alcaligenes* et autres germes Gram négatif - Par l'apparition de colonies incolores ou transparentes et un milieu orange.

7. Mode opératoire selon la revendication 6, dans lequel lorsqu'on utilise X-gal et MUG comme seuls composants d'un mélange qui donnent la couleur ou la fluorescence, l'identification des coliformes totales est réalisée grâce à la couleur bleu verdâtre des colonies, et en particulier, pour *E*. *coli,* également grâce à sa fluorescence bleue.
